# EUROPEAN PATENT SPECIFICATION

(11) **EP 2 564 774 B1**
(45) Date of publication and mention of the grant of the patent: **24.12.2014**
(21) Application number: 11179842.7
(22) Date of filing: 02.09.2011
(51) Int. Cl.: A61B 5/11

(54) **Method for evaluating the work load of an operator in a cycle of manual operations**
Methode zur Ermittlung der Arbeitsbelastung eines Operators während eines Zyklus von manuellen Operationen
Méthode pour l'évaluation de la charge de travail d'un opérateur dans un cycle d'opérations manuelles

(43) Date of publication of application: 06.03.2013
(73) Proprietor: Fiat Group Automobiles S.p.A., 10135 Torino (TO) (IT)
(72) Inventor: D'Aprile, Paola, 10043 Orbassano (Torino) (IT); Lamacchia, Sebastiano, 10043 Orbassano (Torino) (IT); Lionello, Davide, 10043 Orbassano (Torino) (IT); Antonini, Bruno, I-10135 Torino (IT)
(74) Representative: Notaro, Giancarlo

(56) References cited:
- US-A- 5 592 401
- US-A1- 2002 167 205
- US-A1- 2003 083 596
- US-A1- 2010 036 288

## Description

The present invention refers to a method for evaluating the work load of an operator during the performance of a cycle of manual operations, in an industrial production. Methods having the features of the preamble of claim 1 are disclosed in US 5 592 401 A, US 2010/036288 A1, US 2002 167205 A1 and US 2003/083596 A1.

Over the last years there has been an ever-growing need for guaranteeing that the manual operations performed by an operator during the work thereof are ergonomically acceptable and that they are not harmful for the operator in question.

Up to date, the evaluation of the physical work load has been based on detection and analysis systems different and not integrated with each other. In particular it has been previously proposed to analyse and monitor the forces to which a given part of the body of the operator, typically the hand, is subjected to during the performance of a given operating cycle, or it was proposed to monitor the oscillation angle of one or more articulations of the body of the operator during the performance of the manual operations provided for in a given work cycle.

The object of the present invention is to provide a method for multiparameter analysis capable of obtaining a reliable evaluation of the work load of the operator according to a correlated detection of the forces and the oscillation angles to which parts of the body of the operator are subjected.

With the aim of attaining such object, the invention is directed to a method as set forth in claim 1.

Electromyography (EMG) is a known technique for studying and analyzing the muscular activity and muscular fatigue. It is for example carried out by means of needle electrodes inserted in the muscles to be monitored. According to such known technique there occurs the registration of the activity of the muscular fibres under various conditions: at rest, during contraction that is voluntary and progressive up to a maximum effort and during a medium considerable contraction. In practice, electromyography verifies the electrical activity related to the muscular contraction.

The idea on which the present invention is based is to use the electromyographic detection for classifying the degree of burden of the work load of the operator defined by the correlated force and oscillation angle values detected on the body of the operator during the performance of the cycle of operations he is intended to perform.

In a typical embodiment, the aforementioned first sensor means are piezoelectric sensors carried by a glove which is worn by the operator, for detecting the forces applied to the hand used during the performance of the cycle of manual operations.

Further characteristics and advantages will be apparent from the description that follows with reference to the attached drawings, provided purely by way of non-limiting example, wherein:
- figure 1 illustrates an operator during the performance of a work cycle,
- figure 2 shows a glove provided with sensors which is worn by an operator so as to detect the forces to which the hand of the operator is subjected during the performance of the operations,
- figure 3 shows an electrogoniometer and an electronic torque meter applied to the articulations of the elbow and the wrist of the operator with the aim of detecting the oscillation angles of such articulations during the performance of the operations,
- figure 4 shows a series of detectors applied to the body of the operator so as to perform an electromyographic detection of the muscular activity of the operator,
- figure 5 is a block diagram of the apparatus used in the method according to the invention, and
- figures 6, 7 are raised and plan schematic views of a work station comprising parts that are quickly adjustable in position and/or configuration for adapting the characteristics of the work station to the characteristics of the operator, according to the results obtained through the method according to the invention.

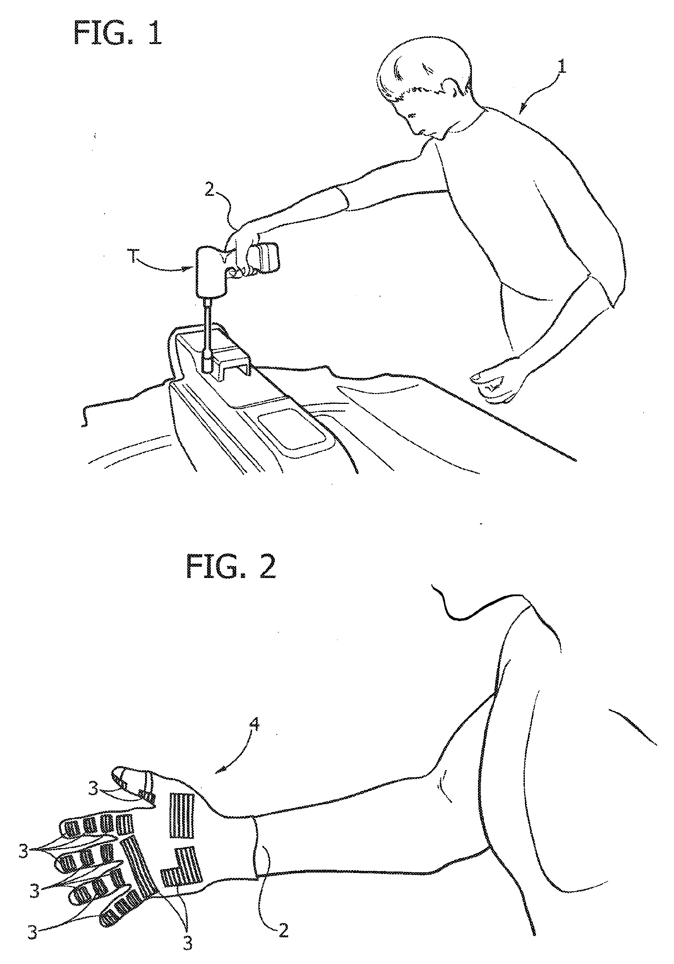

The method according to the invention aims at providing a reliable detection of the work load to which an operator 1 (figure 1) is subjected during the performance of a cycle of operations in a work station of an industrial plant. Figure 1 illustrates the operator 1 gripping a tool T with the hand 2 (in the illustrated example the tool T is constituted by a screw runner). The cycle of operations may successively include operations that are carried out using tools and operations that are performed directly by the operator and which may include imparting slight knocks - using the hand - to elements that should be assembled to each other, manually transfer the pieces from one position to another, supporting the weight thereof, or any other operation that requires an activity of the muscles articulations of the body of the operator.

As mentioned previously at the beginning of the present description, in the past some parameters indicating the work load of the operator were monitored, but always separately with respect to each other. The method according to the invention instead aims at identifying a criterion capable of allowing providing a classification of the work load considering the simultaneous existence of a rotation of a given articulation of the body of the operator (for example the wrist or elbow) and a force applied using such part of the body by the operator. It is clear that if there are limits to the forces that the operator is required to bear in the work thereof, a careful ergonomic analysis requires that such limits be lowered when the forces are supported with the simultaneous presence of an oscillation of the involved articulation.

For such purpose, the method according to the invention uses both sensor means for detecting the forces applied to the body of the operator during the performance of the cycle of manual operations and sensor means for detecting the oscillation angles of the articulations of the body involved by a predefined movement.

Figure 2 shows, by way of example, force sensor means constituted by a glove 4 which is worn on the hand 2 of the operator and which incorporates a plurality of piezoelectric sensors 3 both on the phalanges of the fingers and in various areas of the palm of the hand, for detecting the forces on such areas of the hand during the performance of the cycle of manual operations.

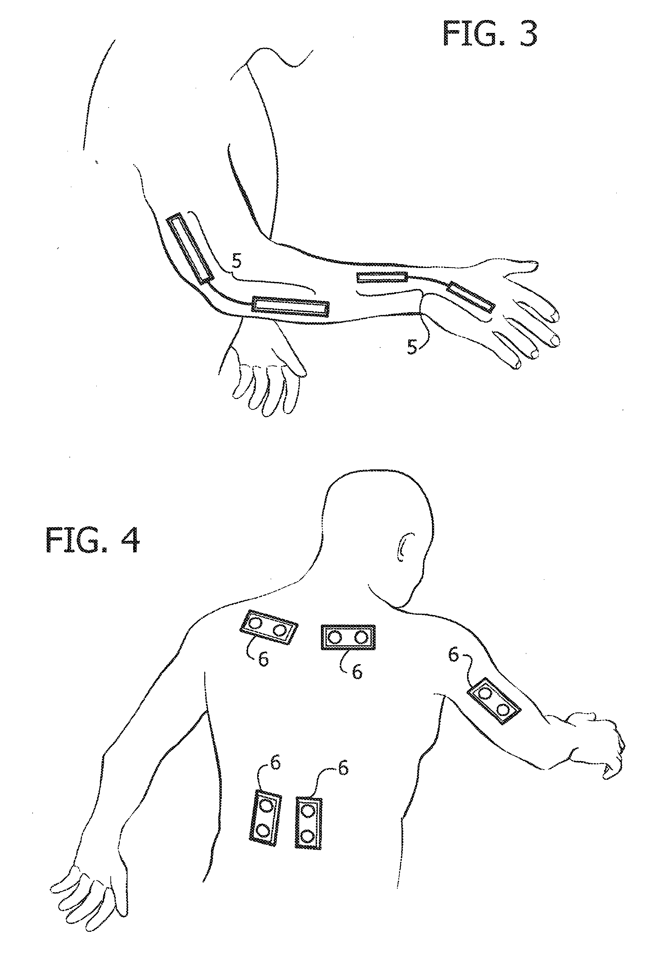

At the same time, as illustrated in figure 3, the method provides for the application of rotation angle sensors at one or more articulations of the body of the operator. Figure 3 shows an electrogoniometer 5 and a torque meter 6 of any per se known type, applied to the articulation of the elbow and to the articulation of the wrist, for detecting the rotation angles at such articulations.

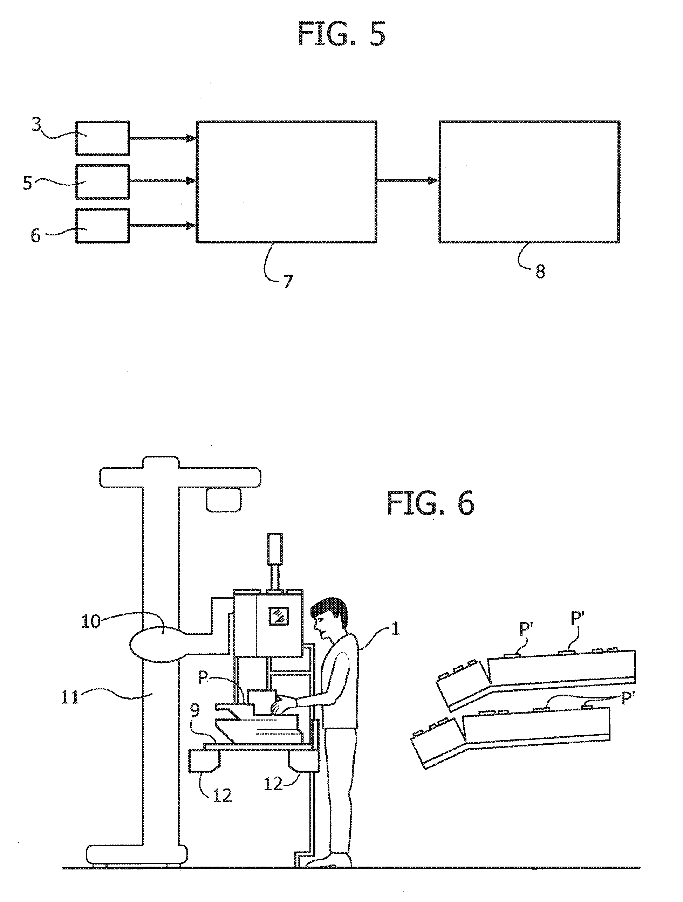

With reference to figure 5, the apparatus used in the method according to the invention comprises an electronic processing and control unit 7 which receives the signals coming from the means 3 for sensing the forces applied to the body of the operator and from the means 5 for sensing the oscillation angles of the articulations of the body of the operator. The work load the operator is subjected to is classified not considering the detected force values considered alone and the detected angle values considered alone, but considering the simultaneous presence of such parameters, i.e. the fact that given forces are applied to given oscillation angles. According to the invention, the parameter used for classifying the work load corresponding to different pairs of force-angle values is constituted by an electromyographic detection which is performed on the body of the operator during the performance of the cycle of manual operations. Electromiography is a per se known technique and the means used for such purpose within the method according to the invention can be of any known type. Therefore, they are not illustrated in detail in the present description, also due to the fact that, considered alone, they do not fall within the scope of protection of the invention, and the elimination of such details from the drawings makes the latter easier to understand. By way of example, figure 4 illustrates a plurality of sensors 6 used for the electromyographic detection.

Thus, as schematically indicated in figure 5, the processing unit 7 receives, besides the signals coming from the force sensors 3 and angle sensors 5, also signals coming from the electromyographic sensors 6. The unit 7 is programmed according to an algorithm which evaluates the various pairs of force-angle values and hence classifies the work load of the operator, using - as parameter for such classification - the electromyographic data corresponding to each pair of force-angle values. The results of such processing are sent to display means 8 of any known type which provide a display of the classification of the work load of the operator thus obtained.

Due to the results that can be obtained through the method according to the invention, a work station can be configured in an ideal manner as a function of the specific characteristics of a given operator, with the aim of reducing the work load thereof to the minimum.

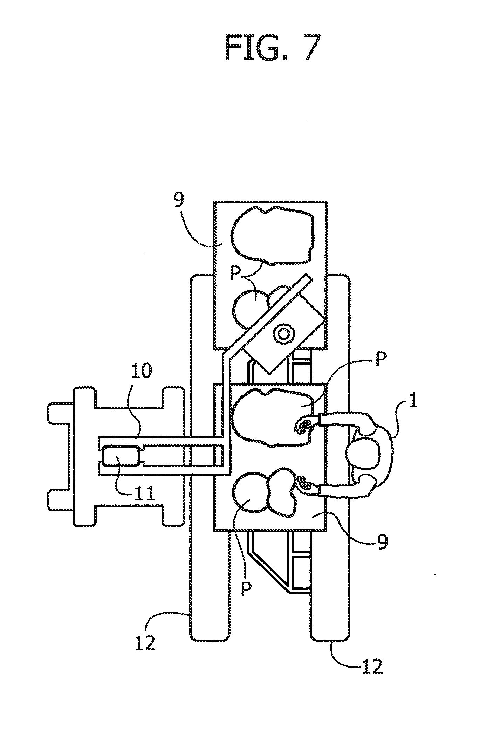

Figures 6, 7 schematically show a work station constituted by various parts which can be quickly adjusted in position and/or configuration to adapt them to the characteristics of the operator. In the illustrated example, the operator 1 is required to perform a series of manual operations on pieces P carried by a pallet 9 which are moved along a line arranged facing the operator, on guides 12. When performing such operations, the operator also interacts with further elements P' (figure 6) arranged behind him. As illustrated, the height of the work plane can be adjusted, in that such work plane is carried by a slide structure 10 moveable vertically on a vertical upright 11. Furthermore, the station may provide for further parts (not illustrated) adjustable in position also in a horizontal direction or according to a circular trajectory around an articulation axis. The station is provided having motor means of any known type (not illustrated) which allow controllably positioning the aforementioned parts of the work station in a desired position according to instructions provided by a control unit. According to the invention, the control unit adjusts the position of the various parts constituting the work stations according to the specific characteristics of the operator found at the work station from time to time. For such purpose, the control unit is provided having means for reading a recording medium of any known type (for example a magnetic card) on which the operator's data is recorded. Hence the processing unit provides for rapidly positioning the various parts of the work station according to the specific characteristics of the operator. The unit is programmed to perform such operation according to a criterion of minimising the work load of the operator considering the data obtained previously through the method forming an object of the present invention, as described above. In other words, an accurate and reliable classification of the work load of the operator, which also considers the fact that when performing given manual operations the operator exerts given forces at given rotation angles of the articulations of the body thereof (in particular the wrist) allows programming a positioning of the various parts of the work station capable of reducing the work load of the operator to the minimum.

Obviously, the angle sensors 5 can be applied for detecting the various types of rotation of the wrist of the operator (i.e. both the oscillation of the hand in the plane of the palm of the hand, and the flexions of the hand in the direction orthogonal to the palm of the hand and the torsions of the wrist).

As clear from the description above, the method according to the invention allows obtaining, for the first time, an evaluation of the work load based on the correlated analysis of the force and angle data with the help of the electromyographic data.

The work station besides automatically adapting the layout of the work station to the specific characteristics of the operator (height, gender, possible lower working capacity) identified by reading an identification electronic card (badge), is designed considering the results of a preliminary analysis of the work load of the operator, performed through the method according to the invention.

Naturally, without prejudice to the principle of the invention, the construction details and the embodiments may widely vary with respect to what has been described and illustrated purely by way of example, without departing from the scope of protection of the present invention as defined by the appended claims.

## Claims

1. Method for adapting a work station where a cycle of manual operations are performed by an operator in order to reduce the work load of the operator to a minimum,
wherein said method comprises the step of evaluating the work load of the operator (1) during the performance of said cycle of manual operations,
wherein said step of evaluating is performed with the aid of:
- first sensor means (3) provided for detecting the forces to which a part of the body of the operator is subjected during the performance of the cycle of manual operations,
- second sensor means (5) provided for detecting the oscillation angles of one or more articulations associated to said part of the body of the operator, during the performance of the cycle of manual operations,
- means (6) provided for performing an electromyographic detection on a part of the body of the operator during the performance of the cycle of manual operations, and
wherein said step of evaluating is further performed by processing pairs of values of said forces and said oscillation angles simultaneously detected by said first and second sensor means (3, 5) during the aforementioned cycle of manual operations, and by classifying the work load for the operator using - as classifying parameter - the electromyographic detection obtained at each of said pairs of values of force and angle,
**characterised in that** said method further comprises the step of recording on a recording medium the characteristics of the operator, including height, gender, possible lower working capacity, and the step of quickly adjusting in position and/or configuration one or more elements (10) forming part of the station on the basis of said recording medium so as to quickly adapting the work station to the characteristics of the operator, in order to reduce his work load to a minimum.

2. Method according to claim 1, **characterised in that** said first sensor means are piezoelectric sensors (3) applied to the body of the operator.

3. Method according to claim 1, **characterised in that** said second sensor means (5) comprise electrogoniometers and electronic torque meters (5) applied to the body of the operator.

4. Method according to claim 1 or 2, **characterised in that** said first sensor means (3) are carried by a glove (4) which is worn by the operator for detecting the forces applied to the hand (2) during the performance of the cycle of manual operations.

## Patentansprüche

1. Verfahren zum Anpassen eines Arbeitsplatzes, an dem ein Zyklus von manuellen Operationen von einem Operator durchgeführt wird, zur Herabsetzung der Arbeitsbelastung des Operators auf ein Minimum,
wobei das Verfahren den Schritt des Ermittelns der Arbeitsbelastung des Operators (1) während des Durchführens des Zyklus von manuellen Operationen umfasst,
wobei der Schritt des Ermittelns mithilfe von Folgendem durchgeführt wird:
- ersten Sensormitteln (3), die zum Erfassen der Kräfte vorgesehen sind, denen ein Teil des Körpers des Operators während des Durchführens des Zyklus von manuellen Operationen ausgesetzt ist,
- zweiten Sensormitteln (5), die zum Erfassen der Schwingungswinkel von einem oder mehreren Gelenken, die dem Teil des Körpers des Operators zugehörig sind, während des Durchführens des Zyklus von manuellen Operationen vorgesehen sind,
- Mitteln (6), die zum Durchführen einer elektromyographischen Messung an einem Teil des Körpers des Operators während des Durchführens des Zyklus von manuellen Operationen vorgesehen sind, und
wobei der Schritt des Ermittelns ferner vorgenommen wird durch Verarbeiten von Paaren von Werten der Kräfte und der Schwingungswinkel, die gleichzeitig von den ersten und zweiten Sensormitteln (3, 5) während des zuvor erwähnten Zyklus von manuellen Operationen erfasst werden, und durch Einteilen der Arbeitsbelastung für den Operator unter Verwendung von - als Einteilungsparameter - der elektromyographischen Messung, die an jedem der Wertepaare aus Kraft und Winkel erhalten wird,
**dadurch gekennzeichnet, dass** das Verfahren ferner den Schritt des Aufzeichnens der Merkmale des Operators einschließlich Größe, Geschlecht, eines möglichen geringeren Arbeitsvermögens auf einem Aufzeichnungsmedium umfasst, und den Schritt des schnellen Anpassens der Position und/oder Anordnung von einem oder mehreren Elementen (10), die einen Bestandteil des Platzes bilden, auf der Grundlage des Aufzeichnungsmediums zum schnellen Anpassen des Arbeitsplatzes an die Merkmale des Operators, damit seine Arbeitsbelastung auf ein Minimum herabgesetzt wird.

2. Verfahren nach Anspruch 1, **dadurch gekennzeichnet, dass** es sich bei den ersten Sensormitteln um piezoelektrische Sensoren (3) handelt, die am Körper des Operators angebracht werden.

3. Verfahren nach Anspruch 1, **dadurch gekennzeichnet, dass** die zweiten Sensormittel (5) Elektrogoniometer und elektronische Drehmomentmesser (5) umfassen, die am Körper des Operators angebracht werden.

4. Verfahren nach Anspruch 1 oder 2, **dadurch gekennzeichnet, dass** die ersten Sensormittel (3) von einem Handschuh (4) getragen werden, den der Operator zum Erfassen der Kräfte trägt, die während des Durchführens des Zyklus von manuellen Operationen auf die Hand (2) einwirken.

## Revendications

1. Procédé pour adapter un poste de travail où un cycle d'opérations manuelles est réalisé par un opérateur afin de réduire la charge de travail de l'opérateur au minimum,
où ledit procédé comprend l'étape d'évaluation de la charge de travail de l'opérateur (1) pendant la réalisation dudit cycle d'opérations manuelles,
où ladite étape d'évaluation est réalisée à l'aide :
- de premiers moyens de détection (3) fournis pour détecter les forces auxquelles une partie du corps de l'opérateur est soumise pendant la réalisation du cycle d'opérations manuelles,
- de deuxièmes moyens de détection (5) fournis pour détecter les angles d'oscillation d'une ou de plusieurs articulation(s) associée(s) à ladite partie du corps de l'opérateur, pendant la réalisation du cycle d'opérations manuelles,
- des moyens (6) fournis pour réaliser une détection électromyographique sur une partie du corps de l'opérateur pendant la réalisation du cycle d'opérations manuelles, et
où ladite étape d'évaluation est en outre réalisée en traitant des paires de valeurs desdites forces et desdits angles d'oscillation détectées simultanément par lesdits premiers et deuxièmes moyens de détection (3, 5) pendant le cycle susmentionné d'opérations manuelles, et en classant la charge de travail pour la opérateur en utilisant - en tant que paramètre de classification - la détection électromyographique obtenue à chacune desdites paires de valeurs de force et d'angle,
**caractérisé en ce que** ledit procédé comprend en outre l'étape d'enregistrement, sur un support d'enregistrement, des caractéristiques de l'opérateur, y compris la taille, le sexe, une éventuelle capacité de travail plus faible, et l'étape d'ajustement rapide en position et/ou en configuration d'un ou de plusieurs élément(s) (10) faisant partie de la station sur la base dudit support d'enregistrement de manière à adapter rapidement le poste de travail aux caractéristiques de l'opérateur, afin de réduire sa charge de travail au minimum.

2. Procédé selon la revendication 1, **caractérisé en ce que** lesdits premiers moyens de détection sont des capteurs piézoélectriques (3) appliqués au corps de l'opérateur.

3. Procédé selon la revendication 1, **caractérisé en ce que** lesdits deuxièmes moyens de détection (5) comprennent des électrogoniomètres et des couplemètres électroniques (5) appliqués au corps de l'opérateur.

4. Procédé selon la revendication 1 ou 2, **caractérisé en ce que** lesdits premiers moyens de détection (3) sont portés par un gant (4) qui est porté par l'opérateur pour détecter les forces appliquées à la main (2) pendant la réalisation du cycle d'opérations manuelles.
